(19) Europäisches Patentamt
European Patent Office
Office européen des brevets

(11) EP 2 264 042 B1

(12) EUROPEAN PATENT SPECIFICATION

(45) Date of publication and mention
of the grant of the patent:
**18.07.2012 Bulletin 2012/29**

(21) Application number: **10175145.1**

(22) Date of filing: **25.07.2008**

(51) Int Cl.:
*C07H 3/02* (2006.01)     *C07H 3/04* (2006.01)
*A23P 1/00* (2006.01)     *A23G 4/10* (2006.01)
*A23L 1/09* (2006.01)     *A23L 1/236* (2006.01)
*A23G 3/34* (2006.01)     *A23G 3/38* (2006.01)
*A23G 3/42* (2006.01)

(54) **Micronization of polyols**

Mikronisierung von Polyolen

Micronisation de polyols

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **27.07.2007 EP 07113374**

(43) Date of publication of application:
**22.12.2010 Bulletin 2010/51**

(62) Document number(s) of the earlier application(s) in accordance with Art. 76 EPC:
**08775360.4 / 2 183 262**

(73) Proprietor: **Cargill, Incorporated
Wayzata, MN 55391 (US)**

(72) Inventors:
• **Stouffs, Robert, Henri, Marcel
I-44100, Ferrara (IT)**
• **Gonze, Michel, Henri, Andre
B-1020, Brussels (BE)**

(74) Representative: **Wilkinson, Stephen John
Stevens, Hewlett & Perkins
1 St. Augustine's Place
Bristol BS1 4UD (GB)**

(56) References cited:
**WO-A-2004/067595**

• **N.N.: "Sanitary Design Micronizer, USDA-accepted Jet Mills" [Online] 2005, STURTEVANT. INC. 348 CIRCUIT STREET, HANOVER, MA 02339 , XP002463036 Retrieved from the Internet: URL:http://www.sturtevantinc.com/micornizer.php> [retrieved on 2007-12-18] * the whole document ***
• **Z. MA; ET AL.: "In-line Particle Size Measurement for Control of Jet Milling" PARTICLE AND PARTICLE SYSTEMS CHARACTERIZATION, vol. 18, 2001, pages 99-106, XP002463035**

EP 2 264 042 B1

**Description**

<u>Technical Field</u>

The current invention relates to micronization of polyols. Polyols with improved properties can be obtained and can be applied in food, feed, cosmetic and pharmaceutical applications.

<u>Background of invention</u>

Polyol powders are prepared according to different technologies. Polyols can be crystallised, freeze-dried, extruded, spray-dried, or steam-agglomerated.

US 4,408,041, US 6,120,612, US 5,932,015 all relate to different processes for crystallising maltitol.

US 5,160,680 describes a method of preparing directly compressible granulated mannitol wherein mannitol powder is subjected to an extrusion treatment.

EP-A-1430887 describes the preparation of inhalable powders for use in the administration of therapeutically-active substances by inhalation. These powders are formed by mixing a coarse powder having an average particle size of 17 to 50 $\mu$m with a fine powder having an average particle size of 2 to 8 $\mu$m.

WO 2004/067595A discloses a process for preparing a granular polyol, e.g. maltitol, involving milling a dried composition to give a particle size of from about 200 to about 2000 $\mu$m.

WO 2006/127560A relates to a chewing gum composition comprising polyols. The average particle size of the polyol composition disclosed ranges from about 30 $\mu$m to about 600 $\mu$m.

It is, further, known to produce a lactose powder having a particle size distribution of 10 to 50 $\mu$m using a micronizer, "Sanitary Design Micronizer, USDA-accepted Jet Mills", 2005 STURTEVANT, INC.

Currently there is a need for a simple, cost-effective process, which allows a polyol of high quality to be obtained.

The current invention provides such a process.

<u>Summary of Invention</u>

The current invention relates to a process for micronizing a solid polyol, said process comprising the following steps:

a) taking a solid polyol,
b) feeding the polyol into a jet mill and applying pressure with nitrogen gas, wherein the pressure applied in the jet mill is in the range of from 2 to 6 bar, and
c) collecting the micronized polyol

and wherein the solid polyol is selected from erythritol, threitol, arabinitol, xylitol, ribitol, allitol, altritol, gulitol, galactitiol, mannitol, sorbitol, talitol, maltitol, isomaltitol, isomalt, lactitol and mixtures of two or more thereof.

The micronized polyols obtained according to the process of the invention are useful in the manufacture of food, feed, cosmetic or pharmaceutical compositions, in particular in the manufacture of chewing gum compositions.

<u>Detailed Description</u>

The micronized polyol obtained according to the process of the invention typically has a particle size distribution ($d_{50}$) of from 20 to 60 $\mu$m, and a flowability below or equal to 5 s/100 g, preferably below 5 s/100 g.

Although there is no fixed definition, the term "micronized" is generally used to describe particles having an average particle diameter of less than 10 microns, usually with the majority of the particles being between 2 and 5 microns. The present invention, however, relates to a different particles size distribution and, in the context of the present invention, a micronized polyol has a particle size distribution ($d_{50}$) of from 20 to 60 $\mu$m.

The test for measuring the flowability is described in the examples.

The micronized polyol obtained by the process of the invention may be compared favourably with the corresponding milled polyol. Although the micronized polyol has a smaller particle size distribution ($d_{50}$) than the corresponding milled polyol, surprisingly its flowability is improved.

The polyol is selected from the group consisting of erythritol, threitol, arabinitol, xylitol, ribitol, allitol, altritol, gulitol, galactitol, mannitol, sorbitol, talitol, maltitol, isomaltitol, isomalt, lactitol, and mixtures thereof.

In a preferred embodiment, the polyol is selected from the group consisting of maltitol, isomalt, mannitol, sorbitol, xylitol, erythritol and mixtures of one or more thereof. In more specific embodiments, the polyol is erythritol or mannitol.

The micronized polyol obtained by the process of the invention typically has a compressibility index equal to or higher than 40. This compressibility index makes it a potential candidate for application in tablets. The compressibility index is described in the examples.

Jet mills encompass any equipment, which allows the micronization of particulate material and, in particular, the micronization of polyols. Typically suitable equipment is one which allows the reduction of the particle size by mechanical methods and is capable of providing the mironized polyol with a particle size distribution ($d_{50}$) of from 20 to 60 $\mu$m. More typical equipment can encompass jet mills, such as spiral jet mills and opposed jet mills. A typical suitable equipment comprises a cylindrical grinding chamber into which a high velocity gas is introduced via jet nozzles situated around the walls of the chamber. A particulate material to be micronized is introduced into the grinding chamber propelled by a pressurized gas and, inside the chamber, is accelerated around the internal walls of the chamber by virtue of the introduction therein of the high velocity gas. The movement of the gas within the chamber results in the creation of a vortex within which the particulate material is entrained. The particles of the particulate material are, thus, caused to undergo repeated collisions between themselves and, as a result, the particle size distribution ($d_{50}$) of the particulate material becomes reduced. The reduced-size particles, i.e. the micronized product, exit the chamber carried by the exhaust gas and, typically, are passed to a suitable cyclone filter.

The particle size distribution of the micronized product is determined largely by the gas pressure in the chamber and the feed rate of the solid particulate material into the grinding chamber.

Micronization is currently used mainly in the industrial sector (in the production of cement or pigments for paints) and in the pharmaceutical industry for producing solid inhalation products. In these uses, the aim is to produce ultra-fine powders (e.g. particle sizes in the range of from 1 to 10 $\mu$m, or even smaller). In order to be able to achieve such small particle sizes, very high gas pressures (>7 bar) are necessary.

The present invention has demonstrated that suitable products are obtained by applying nitrogen gas.

The present invention is concerned with producing micronized powders of polyols which are particularly useful in the manufacture of edible products, such as food or feed compositions, or of cosmetic or pharmaceutical formulations. Such applications require the micronized polyols to have a particulate size distribution ($d_{50}$) preferably from 20 to 60 $\mu$m. In order to achieve such particle sizes, moderate gas pressures in the range of from 2 to 6 bar are used. The feed polyol whose particle size is reduced according to the process of the invention typically has a starting particle size distribution ($d_{50}$) in the range of from 50 to 500 $\mu$m.

In the present invention, we have achieved good results using either a Micronet M100 jet mill from Nuova Guseo S.r.l. or a Micronizer Jet Mill from Sturtevant Inc. In carrying out the present invention, a dry particulate polyol is typically fed into the grinding chamber of the jet mill through a Venturi injector and conveyed by a pressurized dry, nitrogen gas. Preferably, the feed rate is in the range of 0.5 to 7 kg/hour (in a 10 cm diameter chamber). The particulate polyol is accelerated into a vortex inside the circular chamber of the jet mill by virtue of the injection into the chamber, of a high velocity, dry nitrogen. The repeated particle-on-particle impact caused under the gas pressure in the chamber of the mill grinds the polyol particles to the desired particle size.

The micronized polyol obtained has excellent properties. For instance, since the particle size reduction is achieved without the external application of heat and without the need to use any processing aids, which are commonly used in the micronizing of particulates, the product is not subjected to contamination. Furthermore, by using dry, inert gases in the jet mill, the micronized product is dry and. thus. not vulnerable to lump formation during storage.

Advantages of micronization over conventional hammer mills include the finding that micronized fine powders show much better flow properties (flowability) and stability at storage (absence of lumps after 3 months storage - without any anti-caking agent addition). Micronization also improves confectionery applications and/or pharmaceutical applications, for example, chewing gum composition or tablets.

A micronized polyol obtained according to the process of the invention, preferably micronized mannitol, is useful in the manufacture of a chewing gum composition. A characteristic of the chewing gum composition comprising micronized mannitol obtained by the process of the invention, is the improved hardness of the chewing gum composition. More specifically, it has a hardness of equal to or higher than 3500 g after 24 hours of production, preferably higher than 4000 g.

The present invention is further illustrated by way of the following examples:

## **Examples**

### Example 1

Crystalline mannitol, having an average particle size of 67 $\mu$m, was fed into a Micronizer having a 10 cm diameter grinding chamber using a flow of dry nitrogen. The feed rate of the crystalline mannitol into the Micronizer was 3.1 kg per hour. Dry nitrogen was injected through nozzles into the chamber to maintain a gas pressure ($P_2$) in the chamber of 2 bar (2 x $10^5$ Pa). A dry, free-flowing micronized mannitol, having consistently an average particle size of 33 $\mu$m, was obtained.

Example 2

The procedure described in Example 1 was repeated except that the crystalline mannitol fed to the Micronizer had an average particle size of 82 μm.

The effect of the feed rate of the crystalline mannitol, at two different particle sizes, is shown in the following Table 1.

Table 1

| Crystalline Mannitol | | | | | |
|---|---|---|---|---|---|
| Inlet Gran, (μm) | Outlet Gran. (μm) | $N_2$ Feed ($P_1$) (bar) | $N_2$ Microniz ($P_2$) (bar) | Screw rate (rpm) | Rate (kg/h) |
| 67 | 33 | 2 | 2 | 30 | 3.1 |
| 67 | 24 | 2 | 2 | 20 | 1.7 |
| 82 | 46 | 2 | 2 | 30 | 3.2 |
| 82 | 26 | 2 | 2 | 20 | 0.6 |

The micronized particles of mannitol obtained according to Examples 1 and 2 above were stored in dry conditions for three months after which time no lump formation in the product was observed. The micronized mannitol particles obtained were suitable for use in the manufacture of chewing gum, both in the preparation of the gum base and in the preparation of the coating of the chewing gum.

Example 3

Using a procedure similar to that described in Example 1 above, other polyols were successfully micronized as shown in the following Table 2.

Table 2

| Other Polyols | | | | | | |
|---|---|---|---|---|---|---|
| Product | Inlet Gran. (μm) | Outlet Gran. (μm) | $N_2$ Feed ($P_1$) (bar) | $N_2$ Microniz ($P_2$) (bar) | Screw rate (rpm) | Rate (kg/h) |
| Crystalline maltitol (C* Maltidex CH 16385) | 180 | 38 | 5 | 5 | 20 | 4.1 |
| Maltitol HP Powder | 200 | 33 | 5 | 5 | 27 | 2.8 |
| Crystalline erythritol (C* Eridex 16954) | 400 | 61 | 4 | 4 | 30 | 6.0 |
| Sorbitol powder (C* Sorbidex S16603) | 220 | 40 | 5.8 | 5.8 | 10 | 1.4 |

The micronized erythritol prepared according to example 3 was further analysed for its flowability and compressibility index (%) according to the test procedures described.

The compressibility index (%) is a measure of several properties of a powder: bulk density, particle size and shape, surface area, moisture content and cohesiveness. All of these can influence the observed compressibility index.

The compressibility index (%) of the samples of micronized polyol according to the present invention was determined according to the following procedure.

100g of a sample of polyol powder was placed in a 250ml volumetric cylinder. The apparent volume (Vo) of the unsettled powder in the cylinder was noted. The cylinder containing the powder sample was then mechanically tapped causing the powder in the cylinder to settle. Tapping was continued until no further volume change, due to settling, was observed. The final volume ($V_f$) of the settled powder in the cylinder, after tapping was concluded, was noted.

Using the observed values Vo and $V_f$, the compressibility index (%) is calculated according to the following equation:

$$\text{compressibility index (\%)} = 100 \times \left( \frac{V_o - V_f}{V_o} \right)$$

An average of three determinations is used.

The flowability of a powder is measured as the rate of flow of the material through an orifice. It can be used only for materials that have some capacity to flow and is not, therefore, useful for cohesive materials.

The flowability (s/100g) of the samples of micronized polyol according to the present invention was determined according to the following procedure.

Apparatus

The apparatus used was a Pharma Test PTG-1 from Pharma Test Apperatebau, Hainburg. The apparatus comprised a flow funnel having, at its bottom, a nozzle (orifice) and provided internally with a stirrer. The flow funnel is suspended vertically above a container provided on a balance. The apparatus provides a choice of orifices of different diameters, e.g. 10mm, 15mm and 25mm. The stirrer can be used to help the powder pass through the nozzle and can be used at speeds from 5 to 25 rpm.

The sample of micronized polyol was placed in the flow funnel. The orifice diameter used in the test procedure was 25mm. The stirrer was operated, in the test procedure, at a speed of 25 rpm. The nozzle was opened and the time for 100g of the sample to flow through the nozzle into the container was noted.

An average of three measurements was used for each sample.

The results obtained for flowability and compressibility index (%) are shown in the following table.

| Sample | Flowability (s/100 g) | Compressibility index (%) |
|---|---|---|
| Erythritol fine milled | 6.5 | 38.8 |
| Micronised erythritol according to invention | 4.8 | 41.1 |

Example 4 - Chewing gum preparation

The following recipe was applied for preparing chewing gum.

Recipe:

| Ingredients | % (commercial base) | Dry Weight (g) |
|---|---|---|
| Chewing Gum Base | 36.30 | 23.60 |
| Sorbitol (Cargill -C*Sorbidex S16603) | 33.70 | 21.91 |
| *micronized mannitol according to invention* | *20* | *13.00* |
| Maltitol (Cargill - C*Maltidex L16303) | 8.50 | 5.53 |
| Mint flavour | 1.50 | 0.98 |
| | | |
| **Total** | **100.00** | **65.00** |

The particle size distribution and the flowability of the micronized mannitol compared with standard mannitol and fine milled standard mannitol is shown in the following table.

| | Mannitol Micronized according to the Invention | Mannitol Standard (16705) | Mannitol standard fine milled |
|---|---|---|---|
| Particle size ($d_{50}$) | 43.71 $\mu$m | 77.21 $\mu$m | 34.17 $\mu$m |
| Flowability Nozzle 25 mm $\phi$ with stirring | 4.6 sec / 100g (Sample measured a year after preparation) | 4.3 sec/100g | 7.4 sec / 100g |

Flowability of the micronized mannitol, after storage in a cabinet at 40% Relative Humidity for one year, was again measured. No deterioration in the flowability of the material, after storage, was observed.

The chewing gum composition was formed into sheets and they were stored at room temperature and in a cabinet at 40% Relative Humidity.

The quality of the chewing gum sheets was further determined by measuring the hardness of the chewing gum sheets. For comparison, sheets made using a composition wherein standard fine milled mannitol was used instead of the micronized mannitol of the invention were also subjected to the testing procedure.

Hardness measurement

**Equipment**: Texture Analyscr Profile TA_XTPlus

**TA_XTPlus Settings:**

Mode: Measure Force in Compression
Option: Return to Start
Pre-Test Speed: 0.8 mm/s
Test Speed: 0.8 mm/s
Post-Test peed: 3.0 mm/s
Distance: 2mm
Trigger Force Type: 30.0g
Data Acquisition Rate: 250pps

**Accessory**: 2mm Cylinder Probe (P/2) using 25kg load cell
Heavy Duty Platform (HDP/90) with blank plate

**Test Set-Up**: Place the Heavy Duty Platform onto the machine base. Position the sample chewing gum sheet on the platform, centrally under the probe, and commence the test.

**Observations**: The probe approaches the sample and once the 30.0g trigger force is attained, a rapid rise in force is observed, as the probe penetrates into the chewing gum sheet. The probe returns to its original starting position when a penetration distance of 2mm from the trigger point is reached. The mean penetration force is measured as an indication of the hardness.

The tests were carried out, at ambient temperature, on samples of chewing gum sheets stored at room temperature (22°C) in an air-conditioned room for 24 hours, one week and one month after preparation. The results are shown below.

| Storage at room temperature/Hardness expressed in (g) | 24 hours after preparation | One week after preparation | one month after preparation |
|---|---|---|---|
| Mannitol stand. Fine milled | 3326 | 5254 | 5641 |
| Mannitol micronised according to invention | 4287 | 5976 | 6558 |

# EP 2 264 042 B1

## Claims

1. A process for micronizing a solid polyol, said process comprising the following steps:

   a) taking a solid polyol,
   b) feeding the polyol into a jet mill and applying pressure with nitrogen gas, wherein the pressure applied in the jet mill is in the range of from 2 to 6 bar,
   c) collecting the micronized polyol

   and wherein solid polyol is selected from erythritol, threitol, arabinitol, xylitol, ribitol, allitol, altritol, gulitol, galactitol, mannitol, sorbitol, talitol, maltitol, isomaltitol, isomalt, lactitol and mixtures of two or more thereof.

2. A process according to claim 1, wherein the solid polyol is selected from maltitol, isomalt, mannitol, sorbitol, xylitol, erythritol and mixtures of two or more thereof.

3. A process according to claim 1, wherein the micronized polyol has an average particle size in the range of 20 to 60 $\mu$m and a flowability below or equal to 5 s/100 g, preferably below 5 s/100 g.

4. A process according to claim 2, wherein the micronized polyol has an average particle size in the range of 20 to 60 $\mu$m and a flowability below or equal to 5 s/100 g, preferably below 5 s/100 g.

## Patentansprüche

1. Verfahren zum Mikronisieren eines festen Polyols, wobei das genannte Verfahren die folgenden Schritte umfasst:

   a) Aufnehmen eines festen Polyols,
   b) Zuführen des Polyols in eine Strahlmühle und Auferlegen eines Drucks mit Stickstoffgas, worin der in der Strahlmühle auferlegte Druck im Bereich von 2 bis 6 bar liegt.
   c) Sammeln des mikronisierten Polyols,

   und worin das feste Polyol aus Folgenden ausgewählt ist: Erythritol, Threitol, Arabinitol, Xylitol, Ribitol, Allitol, Altritol, Gulitol, Galactitol, Mannitol, Sorbitol, Talitol, Maltitol, Isomaltitol, Isomalt, Lactitol und Mischungen aus zweien oder mehreren davon.

2. Verfahren nach Anspruch 1, worin das feste Polyol aus Folgenden ausgewählt ist: Maltitol, Isomalt, Mannitol, Sorbitol, Xylitol, Erythritol und Mischungen aus zweien oder mehreren davon.

3. Verfahren nach Anspruch 1, worin das mikronisierte Polyol eine durchschnittliche Partikelgröße im Bereich von 20 bis 60 $\mu$m und eine Fließfähigkeit unter oder gleich 5 s/100 g, bevorzugt unter 5 s/1000 g aufweist.

4. Verfahren nach Anspruch 2, worin das mikronisierte Polyol eine durchschnittliche Partikelgröße im Bereich von 20 bis 60 $\mu$m und eine Fließfähigkeit unter oder gleich 5 s/100 g, bevorzugt unter 5 s/100 g aufweist.

## Revendications

1. Processus de micronisation d'un polyol solide, ledit processus comprenant les étapes suivantes consistant à :

   a) prendre un polyol solide,
   b) amener le polyol dans un broyeur à jet et appliquer une pression avec du gaz d'azote, dans lequel la pression appliquée dans le broyeur à jet est dans la gamme de 2 à 6 bars,
   c) recueillir le polyol micronisé

   et dans lequel le polyol solide est sélectionné parmi de l'érythritol, du thréitol, de l'arabinitol, du xylitol, du ribitol, de l'allitol, de l'altritol, du gulitol, du galactitol, du mannitol, du sorbitol, du talitol, du maltitol, de l'isomaltitol, de l'isomalt, du lactitol et des mélanges d'au moins deux de ceux-ci.

2. Processus selon la revendication 1, dans lequel le polyol solide est sélectionné parmi le maltitol, l'isomalt, le mannitol, le sorbitol, le xylitol, l'érythritol et des mélanges d'au moins deux de ceux-ci.

3. Processus selon la revendication 1, dans lequel le polyol micronisé possède une taille de particule moyenne dans la gamme de 20 à 60 $\mu$m et une aptitude à l'écoulement inférieure ou égale à 5 s/100 g, de préférence inférieure à 5 s/100 g.

4. Processus selon la revendication 2, dans lequel le polyol micronisé possède une taille de particule moyenne dans la gamme de 20 à 60 $\mu$m et une aptitude à l'écoulement inférieure ou égale à 5 s/100 g, de préférence inférieure à 5 s/100 g.

**EP 2 264 042 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4408041 A
- US 6120612 A
- US 5932015 A
- US 5160680 A

- EP 1430887 A
- WO 2004067595 A
- WO 2006127560 A

**Non-patent literature cited in the description**

- Sanitary Design Micronizer, USDA-accepted Jet Mills. STURTEVANT, INC, 2005

9